# EUROPEAN PATENT SPECIFICATION

(11) **EP 1 856 102 B1**
(45) Date of publication and mention of the grant of the patent: **17.06.2009**
(21) Application number: 05708486.5
(22) Date of filing: 28.02.2005
(51) Int. Cl.: C07D 403/06, C07D 207/16, C07D 211/60, A61K 31/445, A61K 31/405

(54) **MEDICAMENTS FOR ALZHEIMER**
MEDIKAMENTE GEGEN ALZHEIMER-KRANKHEIT
MEDICAMENTS CONTRE LA MALADIE D`ALZHEIMER

(43) Date of publication of application: 21.11.2007
(73) Proprietor: Tseti, Ioulia, 145 61 Kifissia (GR)
(72) Inventor: KOUROUNAKIS, Panos, GR-546 55 Thessaloniki (GR); DOULGKERIS, Christos, GR-542 49 Thessaloniki (GR); GALANAKIS, Dimitrios, GR-552 36 Thessaloniki (GR); KOUROUNAKIS, Angeliki, GR-151 27 Melissi, Attiki (GR)
(74) Representative: Kilimiris, Constantinos
(86) International application number: PCT/GR2005/000005
(87) International publication number: WO 2006/090192

(56) References cited:
- EP-A- 0 333 522
- US-A- 4 123 544
- US-A- 5 332 735
- STEINER, JOSEPH P. ET AL: "Neurotrophic immunophilin ligands stimulate structural and functional recovery in neurodegenerative animal models" PROCEEDINGS OF THE NATIONAL ACADEMY OF SCIENCES OF THE UNITED STATES OF AMERICA , 94(5), 2019-2024 CODEN: PNASA6; ISSN: 0027-8424, 1997, XP002348741 cited in the application

## Description

The present invention relates to compounds for the treatment of neurodegenerative diseases, like Alzheimer's disease and related pathological conditions.

Alzheimer's disease (AD) is the most common neurodegenerative disorder, characterised by the progressive deterioration mainly, but not only, of cognitive functions. The initiating molecular events leading to AD are not fully understood, and the pathobiochemical mechanisms involved are highly complex. Nevertheless, brain inflammation^{1,2} oxidative stress^{3,4} and massive loss particularly of cholinergic neurons are detected in all AD brains. Recent studies suggest that the use of non-steroidal anti-inflammatory drugs (NSAIDs) may decrease the risk of developing AD⁵.
¹ Rogers J et al, "Inflammation and Alzheimer's disease", 2000, Neurobiology of Aging, 21, 383-421
² McGeer P, McGeer E, "Inflammation, autotoxicity and Alzheimer's disease", 2001, Neurobiology of Aging, 22, 799-809
³ Behl C, "Alzheimer's disease and oxidative stress: implications for novel therapeutic approaches", 1999, Progress in Neurobiology, 57, 301-323
⁴ Smith M et al, "Oxidative stress in Alzheimer's disease", 2000, Biochimica et Biophysica Acta, 1502, 139-144
⁵ Breitner J, Zandi P, "Do NSAIDs prevent Alzheimer's disease? And if so, why? The epidemiological evidence", 2001, Neurobiology of Aging, 22, 811-817

An aim of the present invention is to provide novel non-steroidal anti-inflammatory derivatives which retain anti-inflammatory action and possess nootropic and antioxidant or neurotrophic or neuroreperative activity while having reduced gastrointestinal toxicity, thus being able to be used for long term treatment.

A first aspect of the present invention is to provide compounds of the general formula-I: 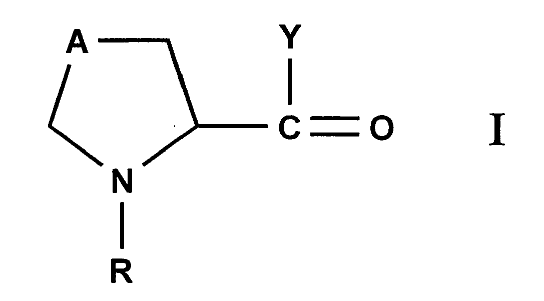
**R** is a non-steroidal anti-inflammatory drug moiety, being selected from indomethacin, naproxen, ibuprofen or ketoprofen,
**A** is a CHOH group or a CH₂ group or a (CH₂)₂ group
**Y** is an antioxidant moiety selected from cysteamine or cysteine ethyl ester or neuroprotective moiety 2-Pyridin-3-yl-alkanol or NGF-inducing moiety 2-methoxy-4-methylphenol.

We chose indomethacin and naproxen of all known NSAIDs because they have the ability to pass the blood brain barrier and reach the target, which in this case is central nervous system. The NSAID and the antioxidant (or neurotrophic or neuroreperative) structures are linked together via a proline or 4-hydroxy-proline moiety. This substituted pyrrolidine structure is derivatised in such a way (amide or ester derivative) to give compounds related to potent nootropic proline derivatives^{6,7}. We chose cysteamine and cysteine (ethyl ester) as antioxidant moieties because they are known good "natural antioxidants", physiologic substances and therefore non-toxic. The 2-methoxy-4-methyl-phenol was used to esterify the NSAID-proline amide because 3,4-dihydroxy-toluene is a known NGF-inducer. These compounds are designed to liberate the above NGF-inducer via biotransformation of the initial molecule, i.e. by hydrolysis and subsequent O-demethylation. It is also possible that the parent compound has NGF-inducing ability ^{8,9}. The 2-Pyridin-3-yl-ethanol has been used since it has been found that the esters of this alcohol acquire neuroreperative properties^{10,11}.
⁶ Gudasheva T. et al, "Synthesis and antiamnesic activity of a series of N-acylprolyl-containing dipeptides", 1996, European Journal of Medicinal Chemistry, 31, 151-157
⁷ EP0486386, US5332735
⁸ Kourounakis A., Bodor N., Simpkins J., 1996, Int. J. Pharm, 141, 239-
⁹ Kourounakis A., Bodor N., Simpkins J., 1997, J. Pharm. Pharmacol., 49, 1-
¹⁰ Steiner J.P. et al, 1997, Proc. Natl. Acad. Sci. USA, 94, 2019-
¹¹ Steiner J.P. et al, 1997, Nature Medicine, 3, 421-

A second aspect of the present invention provides a method for producing a compound of the invention as described above having the general formula I.

A third aspect of the present invention relates to the use of compounds of the present invention (i.e. having the general formula I) in the treatment of Alzheimer's disease or other related neurodegenerative disorders.

A fourth aspect of the present invention provides the use of compounds of the present invention in the preparation of a pharmaceutical for the treatment or retardation of the progress of Alzheimer's disease or other neurodegenerative disorders. Furthermore, the new compounds can be used in cases where inflammation or oxidative stress of CNS is diagnosed or suspected.

A fifth aspect of the present invention provides a pharmaceutical composition containing as active ingredient at least one compound of general formula I.

In accordance with the present invention, the compositions provided may be administered to human individuals or used as a veterinary medicine, particularly for other mammals.
Administration is preferably in a "therapeutically effective amount", this being sufficient to show benefit to a patient. Such benefit may be at least improvement of at least one symptom. The actual amount administered, the rate and time-course of administration, will depend on the nature and severity of what is being treated. Prescription of treatment, e.g. decisions on dosage etc, is within the responsibility of general practitioners and other medical doctors.

Pharmaceutical compositions according to the present invention, and for use in accordance with the present invention, may comprise, in addition to active ingredient, a pharmaceutically acceptable excipient, carrier, buffer, stabilizer or other materials well known to those skilled in the art. Such materials should be non-toxic and should not interfere with the efficacy of the active ingredient. The precise nature of the carrier or other material will depend on the route of administration, which may be oral, nasal, rectal or parenteral.

Pharmaceutical compositions for oral administration may be in tablet, capsule, powder or liquid form. A tablet may comprise a solid carrier such as gelatin or an adjuvant. Liquid pharmaceutical compositions generally comprise a liquid carrier such as water, petroleum, animal or vegetable oils, mineral oil or synthetic oil. Physiological saline solution, dextrose or other saccharide solution or glycols such as propylene glycol or polyethylene glycol may be included.

For intravenous, intramuscular, cutaneous or subcutaneous injection, or injection at the site of affliction, the active ingredient will be in the form of a parenterally acceptable aqueous solution which is pyrogen-free and has suitable pH, tonicity and stability. Those of relevant skill in the art are well able to prepare suitable solutions using, for example, isotonic vehicles such as Sodium Chloride Injection, Ringer's Injection, Lactated Ringer's Injection. Preservatives, stabilizers, buffers, antioxidants and/or other additives may be included, as required.

### Experimental section

Materials: all chemicals are of the highest commercially available purity.
2-Thiobarbiturate acid and diagnostic kits for total cholesterol, LDL and triglycerides determination are purchased from Sigma Co (St Louis MO).
All other reagents are purchased from Aldrich-Chemicals (Steinheim, Germany).
For the in-vivo experiments Fischer-344 rats are used.

Synthesis: melting points (mp) are obtained on a MEL-TEMP II (Laboratory Devices) apparatus and are uncorrected.
Infrared spectra are recorded on a Perkin Elmer 597 infrared spectrophotometer. Proton nuclear magnetic resonance (¹H NMR) are obtained with a Bruker AW 400 MHz spectrometer.
Chemicals shifts are reported in parts per million (δ) relative to tetramethylsilane (TMS) and signals are given as following: s, singlet; d, doublet; t, triplet; m, multiplet. Elemental analyses are performed with a Perkin Elmer 2400 CHN analyzer.

### General procedure for the synthesis of the novel compounds

### Preparation of indomethacin and naproxen chloride:

The NSAID (10 mmol) is dissolved in dichloromethane (ca 100 ml), the solution is cooled in ice, oxalyl chloride (30 mmol) is added dropwise and stirred at 0 °C for 30 minutes. Then it is stirred at room temperature 3 more hours. The solvent and the excess of oxalyl chloride are evaporated under pressure to give a yellow solid (chloride of indomethacin) or a white solid (chloride of naproxen). They are used directly without any further purification.
Yield ca 96%.

### Preparation of indomethacin and naproxen amides with proline:

L-proline (10mmol) is dissolved in equimolar quantity of a 2N sodium hydroxide solution. The solution is cooled at 0°C, the chloride of NSAID (10mmol) is then added while a 4N solution of sodium hydroxide is added dropwise keeping the solution alkaline and at 0°C. After the addition of the NSAID chloride the reaction mixture is kept at room temperature and stirred for one more hour. The alkaline solution is washed with ethyl acetate (3 x 40 ml), the aqueous phase is acidified (pH - 3) with 1N hydrochloric acid and extracted with chloroform (3 x 50 ml), the combined organic extracts are washed with water (2 x 15 ml) and dried with anhydrous magnesium sulphate, filtered and the organic solvent evaporated in vacuo to give the amide of the NSAID with proline (brown-yellow solid in the case of indomethacin and white solid in the case of naproxen).
Yield ca 87%
In the case of the amide of naproxen with 4-hydroxy-proline, the liberated crystalline product, after acidifying the aqueous alkaline solution of the amide, is filtered off, washed with cold water and dried over phosphorus pentoxide in vacuo.
Yield ca 85%

### Synthesis of the amide of amide of indomethacin or naproxen with proline or 4-hydroxy-proline with cysteamine or cysteine ethyl ester:

The NSAID amide with proline or 4-hydroxy-proline (10mmol) is dissolved in dry dimethylformamide (ca 10 ml) (under nitrogen) and 1,1-carbonyldiimidazole (CDI) (10,4 mmol) is added under stirring at 0°C. When the liberation of gas subsided the reaction mixture was left at room temperature under stirring until the completion of the liberation of gas (ca 1 h). The addition of the hydrochloric salt of cysteamine or cysteine ethyl ester (10,1 mmol) follows. The reaction mixture is left under stirring for 24 h. Water is then added dropwise until no more oil is separated, which is extracted by chloroform, the combined organic extracts are dried (MgSO₄) and the solvent is evaporated under reduced pressure. The residue, a yellow oil, is purified by flash chromatography (Silica gel, Pet. Ether/Ethyl acetate).
Yield ca 30-60%

### Synthesis of the ester of amide of indomethacin or naproxen with proline with 2-methoxy-4-methyl-phenol:

The NSAID amide with proline (10mmol) and the 2-methoxy-4-methyl-phenol (40mmol) are dissolved in dry dichloromethane, 1,3 dicyclohexylcarbodiimide (DCC) (20 mmol) and 4-dimethylaminopyridine (DMAP) (1.88 mmol) are added and the mixture is left under stirring for 36 h. The mixture is filtered and the filtrate is washed with an aqueous solution of Na₂CO₃ (10%). The aqueous phase is extracted with dichloromethane (3 x 60), the combined organic extracts are dried (MgSO₄), filtered and the solvent is evaporated under reduced pressure. The residue, a yellow oil, is purified by flash chromatography (Silica gel, Pet. Ether/Ethyl acetate).
Yield ca 40-50%

### EXAMPLES

### Synthesis of compounds 1 to 3 (indomethacin derivatives):

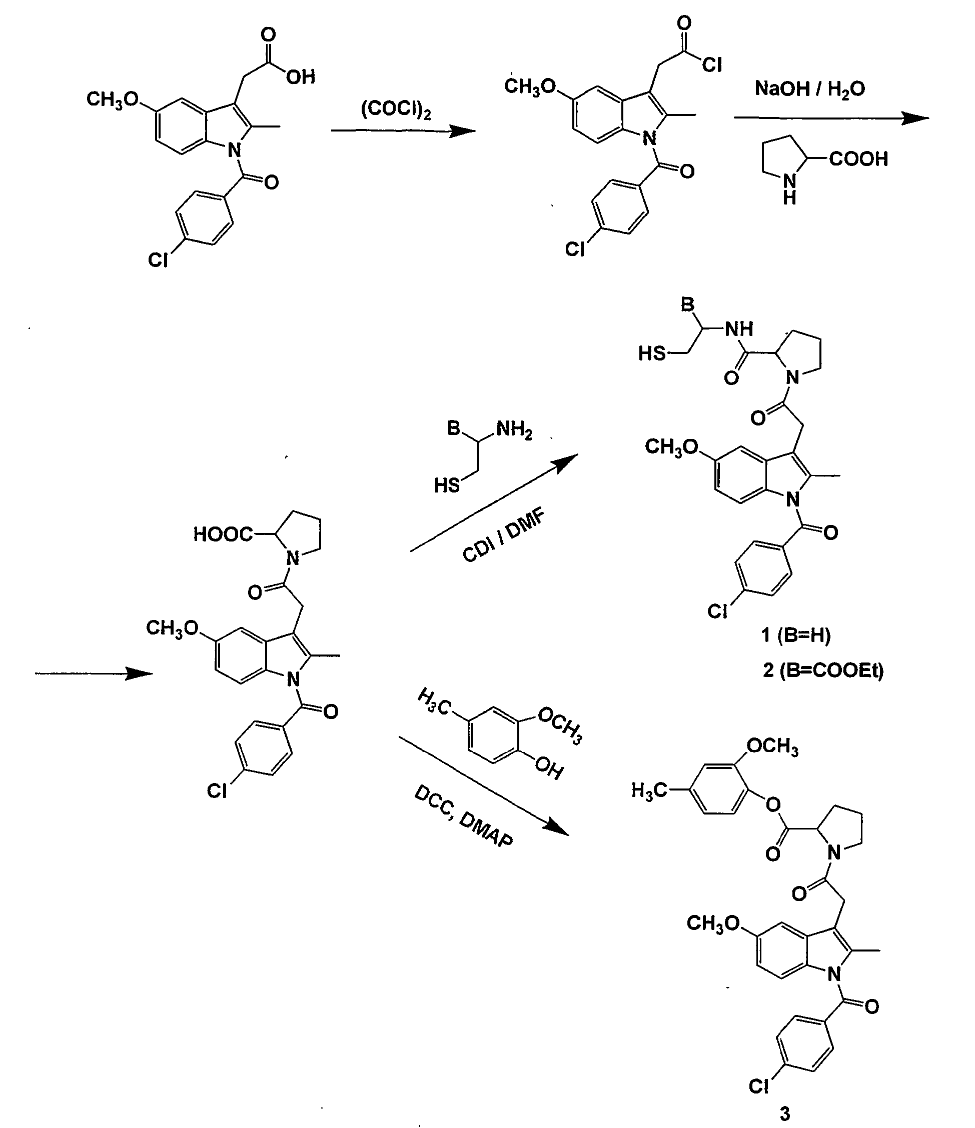

### Synthesis of compounds 4 to 9 (naproxen derivatives):

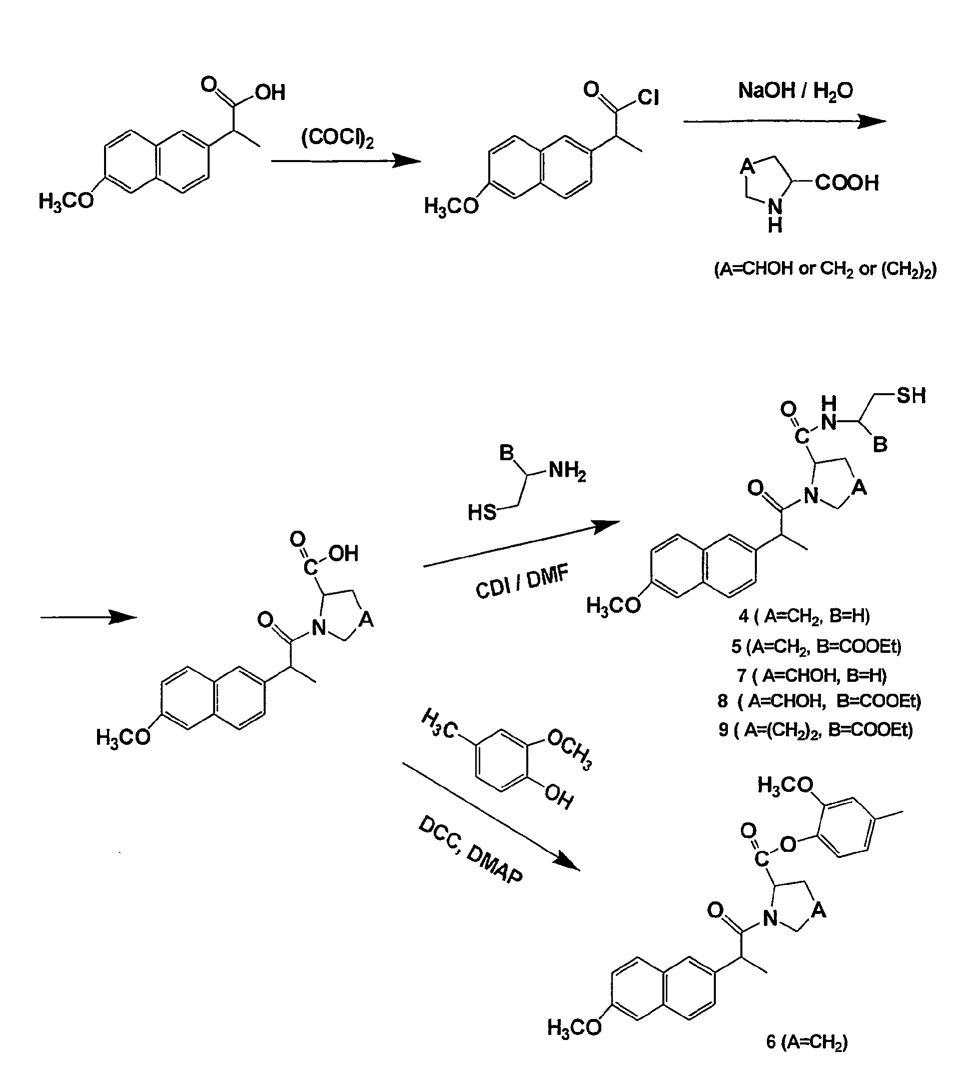

### Compound 1

### 1-{2-[1-(4-Chloro-benzoyl)-5-methoxy-2-methyl-1-H-indol-3-yl]-acetyl}-pyrrolydine-2-carboxylic acid (2-mercapto-ethyl) amide:

Yellow solid. mp: 136-140°C
Analysis for C₂₆H_{Z8}ClN₃O₄S:
Calculated: C: 60.75 H: 5.49 N: 8.17
Found: C: 60.55 H: 5.68 N: 8.51

### Compound 2

### 2-[(1-{2-[1-(4-Chloro-benzoyl)-5-methoxy-2-methyl-1-H-indol-3-yl]-acetyl}-pyrrolidine-2-carbonyl)-amino]-3-mercapto-propionic acid ethyl ester:

Yellow solid. mp: 108-111°C
Analysis for C₂₉H₃₂ClN₃O₆S:
Calculated: C: 59.43 H: 5.50 N: 7.17
Found: C: 59.00 H: 5.56 N: 7.43

### Compound 3

### 1-{2-[1-(4-Chloro-benzoyl)-5-methoxy-2-methyl-1-H-indol-3-yl]-acetyl}-pyrrolidine-2-carboxylic acid-2-methoxy-4-methyl-phenyl ester:

Yellow solid
Analysis for C₃₂H₃₁ClN₂O₆:
Calculated: C: 66.84 H: 5.43 N: 4.87
Found: C: 66.74 H: 5.75 N: 5.27

### Compound 4

### 1-[2-(6-methoxy-naphthalen-2-yl)-propionyl]-pyrrolidine-2-carboxylic acid-(2-mercapto-ethyl) amide

White solid. mp: 151-152°C
Analysis for C₂₁H₂₆N₂O₃S:
Calculated: C: 65.26 H: 6.78 N: 7.25
Found: C: 65.19 H: 7.11 N: 7.24

### Compound 5

### 3-mercapto-2-({1-[2-(6-methoxy-naphthalen-2-yl)-propionyl]-pyrrolidine-2-carbonyl}-amino-propionic acid ethyl ester

Yellow oil
Analysis for C₂₄H₃₀N₂O₅S:
Calculated: C: 62.86 H: 6.59 N: 6.10
Found: C: 62.60 H: 6.31 N: 5.71

### Compound 6

### 1-[2-(6-methoxy-naphthalen-2-yl)-propionyl]-pyrrolidine-2-carboxylic acid 2-methoxy-4-methyl phenyl ester

White solid
Analysis for C₂₇H₂₉NO₅:
Calculated: C: 71.41 H: 6.68 N: 2.94 (with 0.3143 C₄H₈O₂)
Found: C: 71.41 H: 6.89 N: 3.14

### Compound 7

### 4-Hydroxy-1-[2-(6-methoxy-naphthalen-2-yl)-propionyl]-pyrrolidine-2-carboxylic acid-(2-mercapto-ethyl) amide

White solid. mp: 139-141°C
Analysis for C₂₁H₂₆N₂O₄S:
Calculated: C: 62.66 H: 6.51 N: 6.96
Found: C: 62.27 H: 6.69 N: 7.20

### Compound 8

### 2-({4-Hydroxy-1-[2-(6-methoxy-naphthalen-2-yl)-propionyl]-pyrrolidine-2-carbonyl}-amino-3-mercapto-propionic acid ethyl ester

White solid
Analysis for C₂₄H₃₀N₂O₆S:
Calculated: C: 60.74 H: 6.37 N: 5.90
Found: C: 60.38 H: 6.39 N: 6.17

### Compound 9

### 3-Mercapto-2-({1-[2-(6-methoxy-naphthalen-2-yl)-propionyl]-piperidine-2-carbonyl}-amino-propionic acid ethyl ester

Yellow oil
Analysis for C₂₅H₃₂N₂O₅S:
Calculated: C: 63.53 H: 6.82 N: 5.92
Found: C: 63.65 H: 7.26 N: 5.51

### Effect of the compounds on the carrageenan-induced inflammation

Edema was induced via *i.d*. injection of carrageenan (0.1 ml of a 2% w/v aqueous solution) into the right hind paw of female Fischer-344 rats (200-220g) (the left paw serving as control). The compounds under study were dissolved or suspended in water containing a few drops of Tween 80 and were administered *i.p*. (0.3mmol/kg) immediately after the carrageenan injection. The mice were euthanazed 3.5 h later, both hind paws were severed above the ankle joint and weighted separately. For each animal the swelling caused by the phlogistic was given as percentage of weight increase of right hind paw in comparison to the uninjected left hind paw, and from that, the percentage of edema suppression in comparison to controls was calculated. The parent anti-inflammatory acids (at the same molar concentrations) were used as reference compounds, in addition to the absolute control. All derivatives showed inhibition of the edema and in some cases their potency was higher than that of the parent compound.

**Table 1: Effect of the test compounds on carrageenan-induced edema of the rat hind paw**

| **Compound** | **% Edema inhibition** |
|---|---|
| Indomethacin | 47.8 |
| **1** | 23 |
| **2** | 20 |
| Naproxen | 45 |
| **4** | 30 |
| **5** | 30 |
| **6** | 20 |
| **7** | 31 |
| **8** | 60 |

### Effect of the compounds on the adjuvant-induced disease (experimental arthritis)

Adjuvant-induced disease (AID) was induced via *i.d.* injection of Freund's complete adjuvant (0.1 ml of FCA) in the foot pad of the right hind paw of female Fischer-344 rats. The compounds under study were dissolved or suspended in water containing a few drops of Tween 80 and were administered *i.p.* (0.3-0.6 mmol/kg) once daily for 14 days. In order to observe the progress of local inflammatory reaction, the change in swelling of paw with adjuvant treatment was evaluated by allocation of points (inflammatory score) on an arbitrary scale, while for the quantification of arthritis the arthritic score was recorded.

Some of the novel compounds suppressed almost totally the arthritis in comparison to non treated AID rats (which developed severe arthritis), while others ameliorated the symptoms of arthritis.

**Table 2: Effect of the test compounds on FCA-induced experimental arthritis in rats**

| **Compound** | **% Inhibition of experimental arthritis** |
|---|---|
| **1** | 50 |
| **2** | 75 |
| **4** | 30 |
| **5** | 100 |

### Ulcerogenic action of the compounds on rats

Equimolar doses of the parent NSAIDs and the respective derivatives were administered s.c. once daily for 4 days to female Fischer-344 rats (160-180g). The used dose was ca LD50 of the parent NSAID. The incidence of perforating gastrointestinal ulcers, body weight change, melena and mortality were recorded 24 h after the last treatment. The results revealed that administration of the parent NSAIDs, over a period of 4 days and at the dose indicated, resulted in a high incidence of perforating intestinal ulcers, as well as incidence of melena. These effects were accompanied by a 50% mortality of the animals and a pronounced reduction in body weight. In contrast, equimolar doses of the new derivatives exhibited no macroscopic evidence of ulceration and all animals survived with an increase in body weight.

**Table 3: Gastrointestinal toxicity of indomethacin, naproxen and some novel derivatives in rats**

| **Compound** | **Dose (µM/kg)** | **Mortality (%)^{a}** | **Perforatin g ulcers** (%)^{b} | **Body weight change^{c}** | **Incidence of melena** |
|---|---|---|---|---|---|
| Indomethacin | 84 | 50 | 80 | -14.9 | + |
| **1** | 84 | 0 | 0 | +1.3 | - |
| **2** | 84 | 0 | 0 | +3.9 | - |
| **3** | 84 | 0 | 0 | +11.1 | - |
| Naproxen | 1350 | 50 | 100 | -15.8 | + |
| **4** | 1350 | 0 | 0 | +3.5 | - |
| **5** | 1350 | 0 | 0 | +1.5 | - |

| | | | | | |
|---|---|---|---|---|---|
| ^{a} (dead/total) x 100 ^{b} % of animals which developed perforating ulcers ^{c} In g/100 body weight | | | | | |

### Evaluation of the antioxidant activity

### I) In vitro lipid peroxidation

Hepatic microsomal fraction from untreated Fischer-344 rats was prepared. The incubation mixture contained heat inactivated (90°C for 90 sec) hepatic microsomal fraction, corresponding to 2.5 mg protein/ml (fmal concentration) or 4 mmol/L fatty acid residues, ascorbic acid (0.2 mmol/L) in Tris-HCl/KCl buffer (50 mmol/L / 150mmol/L, pH 7.4) and various concentrations (1-0.05 mmol/L) of the test compounds dissolved in dimethylsulfoxide (DMSO). The reaction was started by the addition of a freshly prepared FeSO₄ solution (10 µM/L) and the mixture was incubated at 37°C for 45 min. Aliquots (0.3mL) from the incubation mixture were taken at various time intervals. Lipid peroxidation was assessed spectrophotometrically (535 against 600 nm) by the determination of the TBA reactive material. Under the above experimental conditions, all compounds, as well as DMSO, were tested and found not to interfere with the assay. Each experiment was performed at least in triplicate.

**Table 4: Concentrations of the test compounds that produce 50% inhibition (IC50) of lipid peroxidation at 45 min**

| **Compound** | **IC₅₀ (µM)** |
|---|---|
| **2** | 390 |
| **5** | 320 |
| **8** | 500 |
| **9** | 122 |

### II) Interaction of the test compounds with the stable free radical DPPH

The test compounds were dissolved in absolute ethanol (analytical grade, iron content was less than 10⁻³ (w/v), added to an equal volume of an ethanolic solution of DPPH (final concentration 0.2 mmol/L) at various concentrations (0.1 and 0.2 mmol/L) and kept at room temperature. Absorbance (517 nm) was recorded at 45 min. Each experiment was performed at least in triplicate.

**Table 5: % Interaction of the test compounds with the stable free radical DPPH**

| **Compound** | **0.2mM** | **0.1mm** |
|---|---|---|
| **2** | 70 | 44 |
| **4** | 78 | 57 |
| **5** | 85 | 65 |
| **7** | 56 | 34 |
| **8** | 75 | 55 |
| **9** | 76 | 46 |

### Hypocholesterolemic and hypolipidemic activity

The hypocholesterolemic and hypolipidemic activity of the test compounds was assessed in vivo by the inhibition of tyloxapol-induced hyperlipidemia (200 mg/kg i.p.) in rats. The novel NSAID derivatives were administered (56µmol/kg, *i.p*., once) to hypercholesterolemic rats and 24 h later the plasma total cholesterol (TC), LDL and triglyceride (TG) concentrations were determined in blood taken from the aorta. The results showed that some of the new derivatives possess significant hypocholesterolemic and hypolipidemic activity.

**Table 6: Effect of the test compounds on plasma total cholesterol, triglyceride and LDL-cholesterol levels of hyperlipidemic rats**

| | **% decrease** | | |
|---|---|---|---|
| **Compound** | **TC** | **LDL** | **TG** |
| **5** | 58** | 55* | 68.7** |
| **7** | 45* | 53.5 * | 51 |
| **8** | 35.5 | 56.5* | 25 |
| **9** | 40 | 52* | 21.3 |

| | | | |
|---|---|---|---|
| * *P* < 0.05; ** *P* < 0.01 vs tyloxapol (student's t-test) | | | |

Talking in consideration all parameters used, we rate our best three compounds in the following order:
Compound **5** is the best mode (the best antiarthritic, antioxidant (DPPH), lipid lowering agent and second best as anti-inflammatory and antioxidant (LP) and non-toxic.
Second and third best compounds are compounds **2** and **8.**

All compounds have been designed to have anti-inflammatory activity. However, compound **8** (third best compound) unexpectedly showed much higher anti-inflammatory activity than the parent NSAID (see Table 1).

Some of the compounds are unexpectedly able to reduce hyperlipidemia. This is a property which is very important for certain types of Alzheimer's disease (vascular dementias) (see Table 6).

Compounds **3** and **6** are expected to induce NGF (nerve growth factor) which is an important factor for the preservation, repair and survival of neurons.

To the best of our knowledge we have not found in the literature the claimed compounds. In particular, we have not found compounds which possess combination of all or most of all biological properties (antioxidant, anti-inflammatory, nootropic, hypolipidemic and with greatly reduced gastrointestinal toxicity) that exist in the molecules of this patent.

It is known that non-steroidal anti-inflammatory drugs have as a common side effect gastrointestinal toxicity. Against expectations, our compounds are advantageous because they are lacking this side effect. Therefore they can be administered for long-term treatments.
The antioxidant moieties used are "natural" compounds existing in the human body and they show low or no toxicity at all. Antioxidant capacity reduces the gastrointestinal toxicity of NSAIDs and therefore it is claimed that they further reduce the gastrotoxicity of the incorporated anti-inflammatory moiety. Furthermore, they reduce the oxidative stress in central nervous system.
In addition, it should be emphasized that a number of AD cases (vascular dementias) can be caused by atherosclerosis. One of the common causes of atherosclerosis is hypercholesterolemia. The synthesized compounds possess hypocholesterolemic properties^{12,13,14,15}.
¹² Hofman A. et al, "Atherosclerosis, apolipoprotein E, and prevalence of dementia and Alzheimer's disease in the Rotterdam study", 1997, Lancet, 349, 151-154
¹³ Drachman D. A. et al, "Statins and the risk of dementia", 2000, The Lancet, 356, 1627-1631
¹⁴ Refolo L. et al, "Hypercholesterolemia accelerates the Alzheimer's amyloid pathology in a transgenic mouse model", 2000, Neurobiology of disease, 7, 321-331
¹⁵ Kalaria R., "The role of cerebral ischemia in Alzheimer's disease", 2000, Neurobiology of Aging 21, 321-330

The invention is defined by the claims.

## Claims

1. Compounds of the general formula-I: 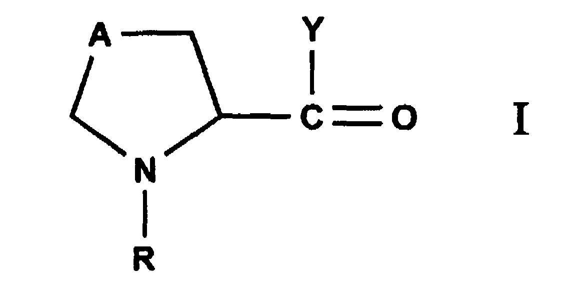
**R** is a non-steroidal anti-inflammatory drug moiety selected from :
i) either the indomethacin or
ii) the propionic acid derivatives consisting of naproxen, ibuprofen or ketoprofen
**A** is a CHOH group of o CH₂ group or a (CH₂)₂ group
**Y** is :
i) an antioxidant moiety selected from cysteamine or cysteine ethyl ester or
ii) the neuroprotective moiety 2-Pyridin-3-yl-alkanol or
iii) the NGF-inducing moiety 2-methoxy-4-methyl-phenol

2. Compound **5** under the formula:
3-mercapto-2-({1-[2-(6-methoxy-naphthalen-2-yl)-propionyl]-pyrrolidine-2-carbonyl}-amino-propionic acid ethyl ester.

3. Compound **2** under the formula:
2-[(1-{2-[1-(4-Chloro-benzoyl)-5-methoxy-2-methyl-1-H-indol-3-yl]-acetyl}-pyrrolidine-2-carbonyl)-amino]-3-mercapto-propionic acid ethyl ester.

4. Compound **8** under the formula:
2-({4-Hydroxy-1-[2-(6-methoxy-naphthalen-2-yl)-propionyl]-pyrrolidine-2-carbonyl}-amino-3-mercapto-propionic acid ethyl ester.

5. Compound **9** under the formula:
3-Mercapto-2-({1-[2-(6-methoxy-naphthalen-2-yl)-propionyl]-piperidine-2-carbonyl}-amino-propionic acid ethyl ester.

6. Compound **1** under the formula:
1-{2-[1-(4-Chloro-benzoyl)-5-methoxy-2-methyl-1-H-indol-3-yl]-acetyl}-pyrrolydine-2-carboxylic acid (2-mercapto-ethyl) amide.

7. Compound **4** under the formula:
1-[2-(6-methoxy-naphthalen-2-yl)-propionyl]-pyrrolidine-2-carboxylic acid-(2-mercapto-ethyl) amide.

8. Compound **3** under the formula:
1-{2-(2-(4-Chloro-benzoyl)-5-methoxy-2-methyl-1-H-indol-3-yl]-acetyl}-pyrrolidine-2-carboxylic acid-2-methoxy-4-methyl-phenyl ester.

9. Compound **6** under the formula:
1-[2-(6-methoxy-naphthalen-2-yl)-propionyl]-pyrrolidine-2-carboxylic acid 2-methoxy-4-methyl phenyl ester.

10. Compound 7 under the formula:
4-Hydroxy-1-[2-(6-methoxy-naphthalen-2-yl)-propionyl]-pyrrolidine-2-carboxylic acid-(2-mercapto-ethyl) amide.

11. Use of anyone of the compounds under anyone of the claims 1-10 for the production of a medicament for the treatment of the disease of Alzheimer or other neurodegenerative disorders or the retardation of the progress of Alzheimer's disease or other neurodegenerative disorders or for the treatment of cases where inflammation or oxidative stress of CNS is diagnosed or suspected.

12. Pharmaceutical composition containing as active ingredient at least one compound of the general formula I of claim 1 or any of the compounds **1** to **9** of claims 2-10.

13. Method of production of the compounds **1** to **3** (indomethacin derivatives):

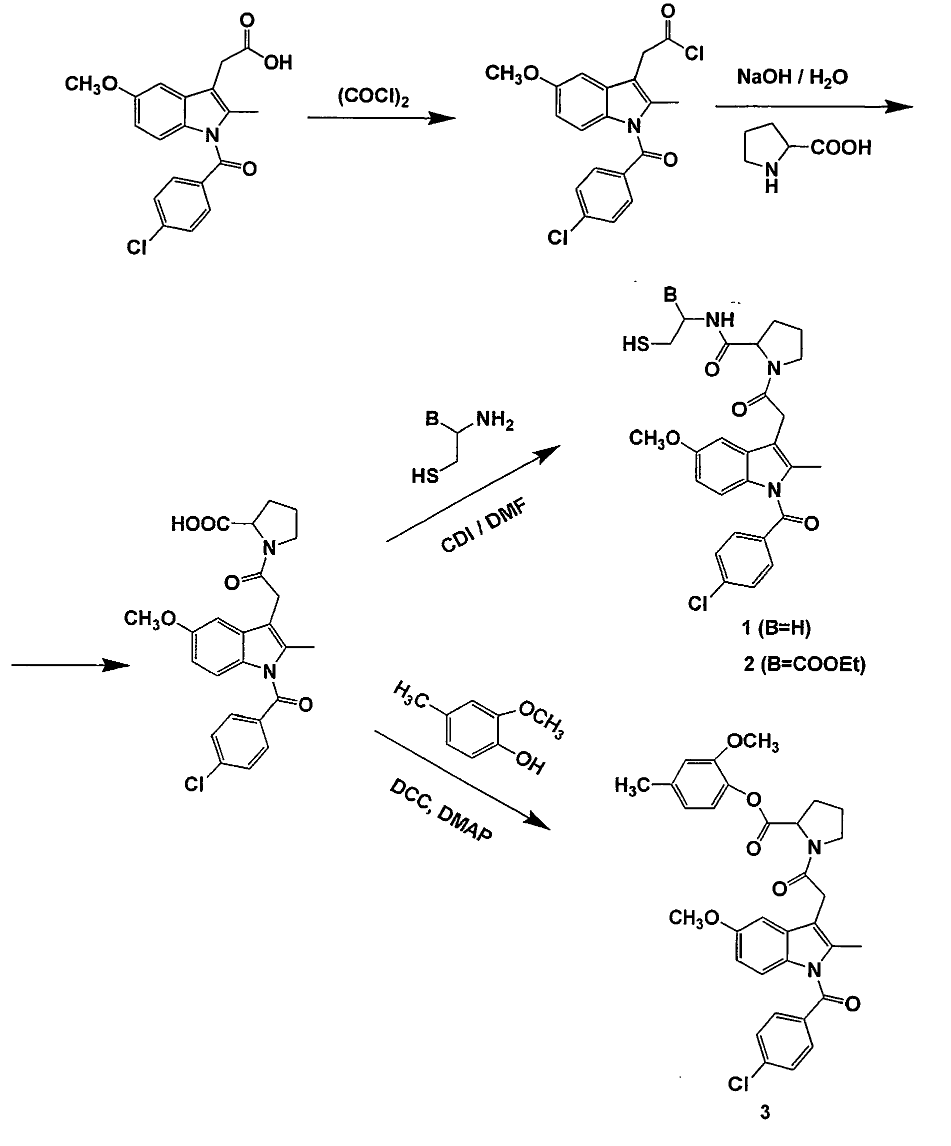

14. Method of production of the compounds **4** to **9** (naproxen derivatives):

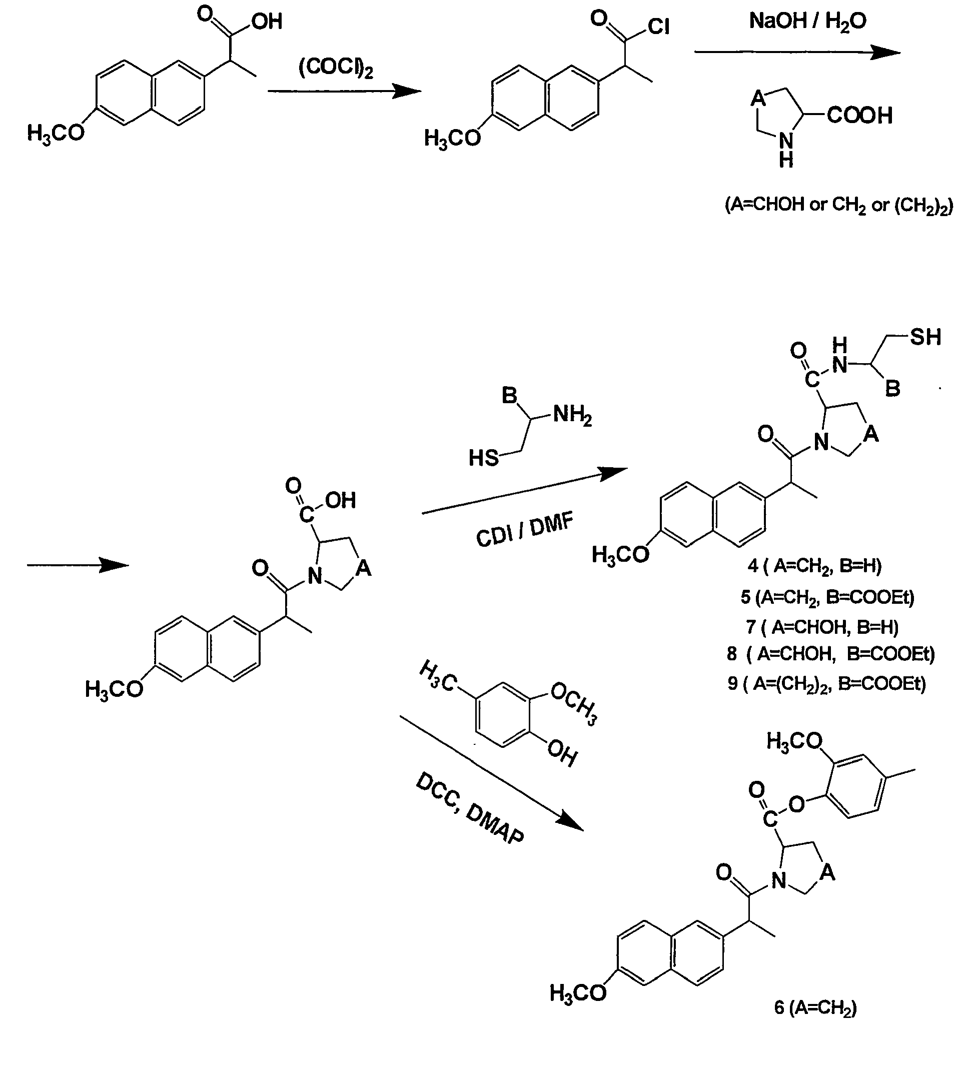

## Patentansprüche

1. Verbindungen der allgemeinen Formel I: 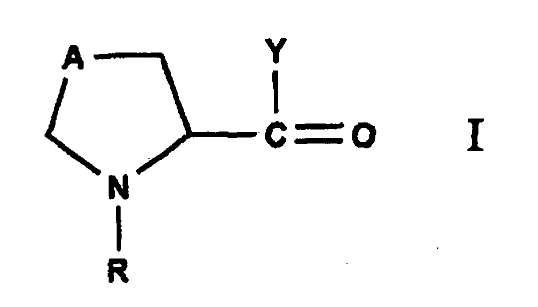
**R** ist ein nichtsteroidaler, antiinflammatorischer Arzneimittelrest ausgewählt aus:
i) entweder dem Indomethacin oder
ii) den Propionsäurederivaten bestehend aus Naproxen, Ibuprofen oder Ketoprofen
**A** ist eine CHOH-Gruppe oder eine CH₂-Gruppe oder eine (CH₂)₂-Gruppe
**Y** ist:
i) ein Antioxidationsmittelrest ausgewählt aus Cysteamin oder Cysteinethylester oder
ii) der neuroprotektive Rest 2-Pyridin-3-yl-alkanol oder
iii) der NGF-induzierende Rest 2-Methoxy-4-methyl-phenol.

2. Verbindung **5** unter der Formel:
3-Mercapto-2-({1-[2-(6-methoxy-naphthalen-2-yl)-propionyl]-pyrrolidin-2-carbonyl}-amino-propionsäureethylester.

3. Verbindung **2** unter der Formel:
2-[(1-{2-[1-(4-Chlor-benzoyl)-5-methoxy-2-methyl-1-H-indol-3-yl]-acteyl)-pyrrolidin-2-carbonyl)-amino]-3-mercapto-propionsäureethylester.

4. Verbindung **8** unter der Formel:
2-({4-Hydroxy-1-[2-(6-methoxy-naphthaten-2-yl)-propionyl]-pyrrolidin-2-carbonly}-amino-3-mercapto-propionsäureethylester.

5. Verbindung **9** unter der Formel:
3-Mercapto-2-({1-[2-(6-methoxy-naphthalen-2-yl)-propionyl]-piperidin-2-carbonyl}-amino-propionsäureethylester.

6. Verbindung **1** unter der Formel:
1-{2-[1-(4-Chlor-benzoyl)-5-methoxy-2-methyl-1-H-indol-3-yl]-acetyl}-pyrrolidin-2-carbonsäure(2-mercapto-ethyl)amid.

7. Verbindung **4** unter der Formel:
1-[2-(6-Methoxy-naphthalen-2-yl)-propionyl]-pyrrolidin-2-carbonsäure-(2-mercapto-ethyl)amid.

8. Verbindung **3** unter der Formel:
1-{2-[1-(4-Chlor-benzoyl)-5-methoxy-2-methyl-1-H-indol-3-yl]-acetyl}-pyrrolidin-2-carbonsäure-2-methoxy-4-methyl-phenylester.

9. Verbindung **6** unter der Formel:
1-[2-(6-Methoxy-naphthalen-2-yl)-propionyl]-pyrrolidin-2-carbonsäure-2-methoxy-4-methylphenylester.

10. Verbindung **7** unter der Formel:
4-Hydroxy-1-[2-(6-methoxy-naphthalen-2-yl)-propionyl]-pyrrolidin-2-carbonsäure-(2-mercapto-ethyl)amid.

11. Verwendung irgendeiner der Verbindungen unter irgendeinem der Ansprüche 1-10 zur Herstellung eines Arzneimittels für die Behandlung der Alzheimer-Krankheit oder anderen neurodegenerativen Erkrankungen oder die Verzögerung des Fortschreitens von Alzheimer-Krankheit oder anderer neurodegenerativer Erkrankungen oder für die Behandlung von Fällen, in denen eine Entzündung oder oxidativer Stress des CNS diagnostiziert ist oder vermutet wird.

12. Pharmazeutische Zusammensetzung, enthaltend als Wirkstoff wenigstens eine Verbindung der allgemeinen Formel I von Anspruch 1 oder irgendeine der Verbindungen **1** bis **9** der Ansprüche 2-10.

13. Verfahren zur Herstellung der Verbindungen **1** bis **3** (Indomethacin-Derivaten):

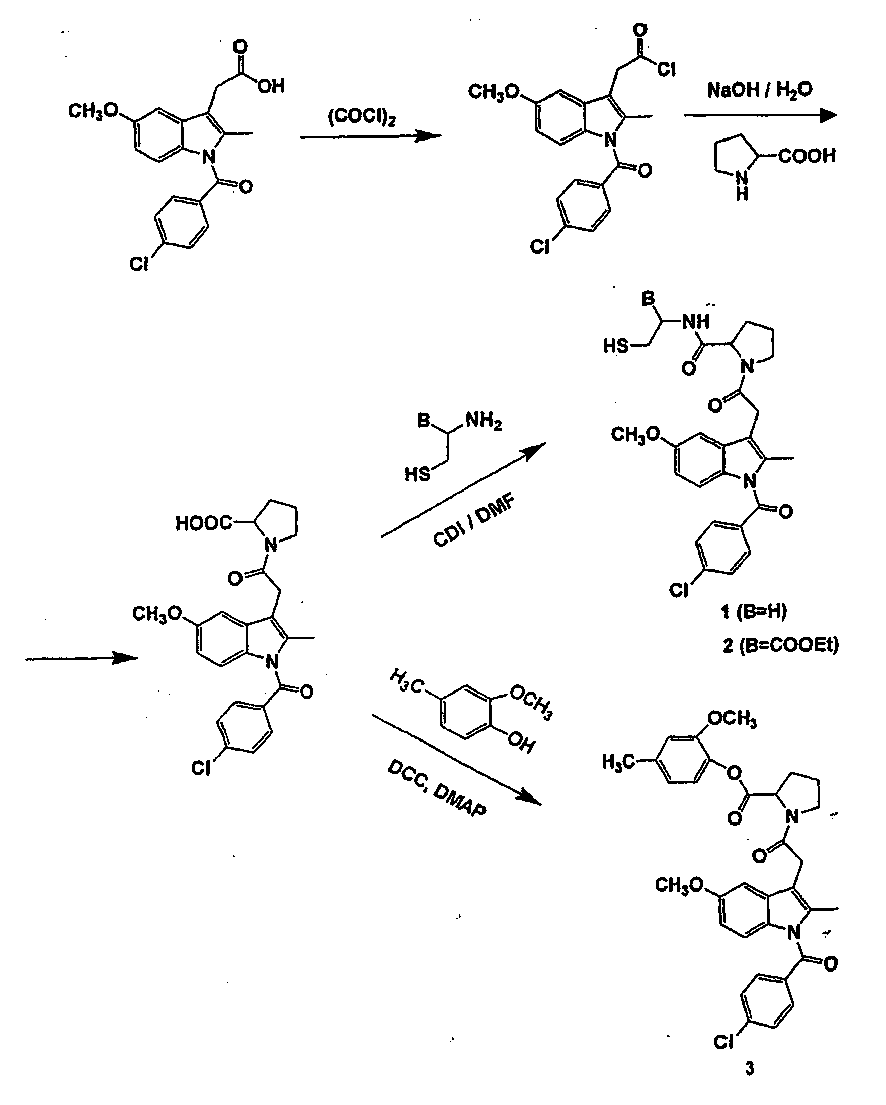

14. Verfahren zur Herstellung der Verbindungen **4** bis **9** (Naproxen-Derivaten):

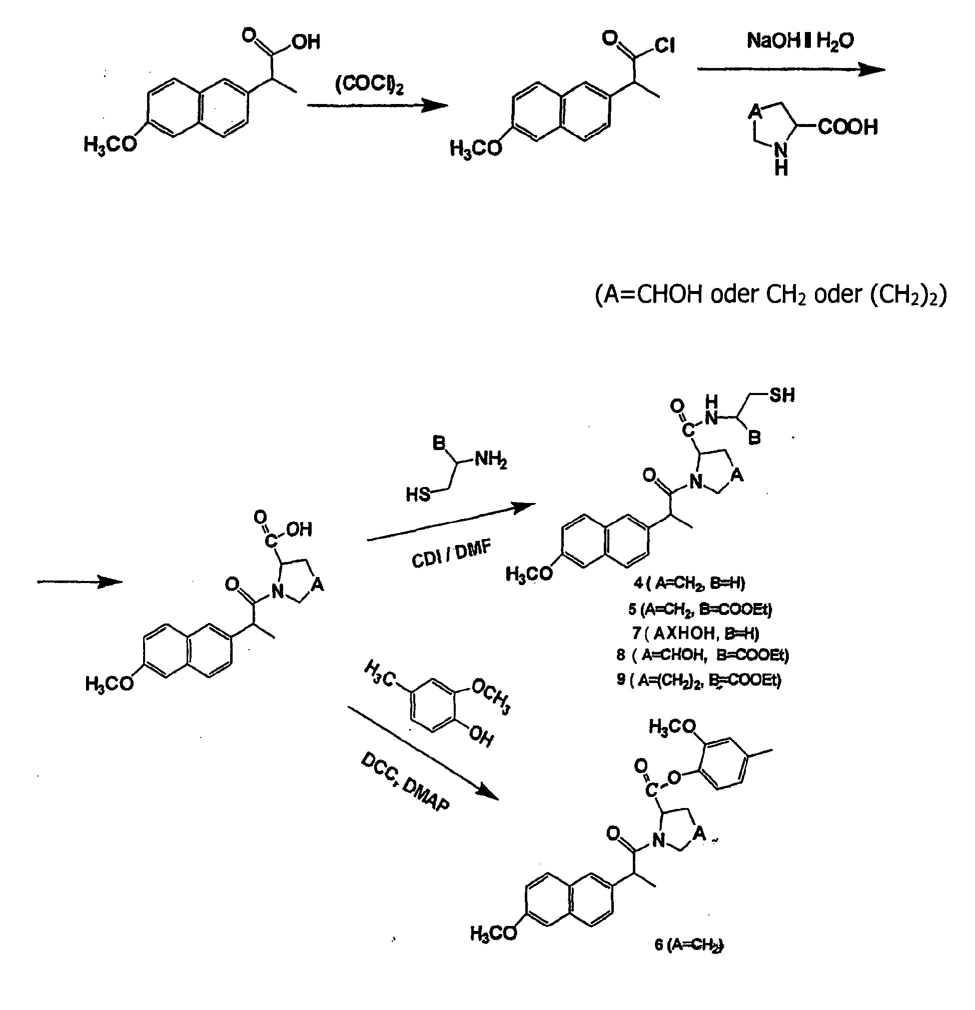

## Revendications

1. Composés de formule générale I : 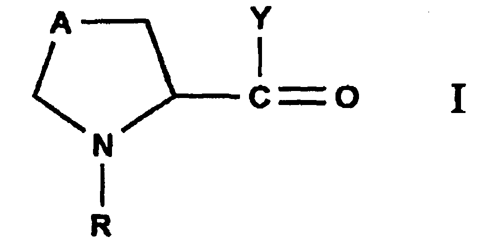
**R** est un fragment de médicament anti-inflammatoire non stéroïdien choisi parmi
i) l'indométhacine ou
ii) les dérivés de l'acide propionique comprenant le naproxène, l'ibuprofène ou le kétoprofène ;
**A** est un groupe CHOH ou un groupe CH₂ ou un groupe (CH₂)₂ ;
**Y** est
i) un fragment antioxydant choisi parmi la cystéamine ou l'ester éthylique de cystéine ou
ii) le fragment neuroprotecteur 2-pyridin-3-yl-alcanol ou
iii) le fragment induisant le NGF 2-méthoxy-4-méthylphénol.

2. Composé 5 ayant la formule :
ester éthylique de l'acide 3-mercapto-2-({1-[2-(6-méthoxy-naphtalén-2-yl)-propionyl]-pyrrolidine-2-carbonyl}-amino-propionique

3. Composé 2 ayant la formule :
ester éthylique de l'acide 2-[(1-{2-[1-(4-chlorobenzoyl)-5-méthoxy-2-méthyl-1H-indol-3-yl]-acétyl}-pyrrolidine-2-carbonyl)-amino]-3-mercapto-propionique.

4. Composé 8 ayant la formule :
ester éthylique de l'acide 2-({4-hydroxy-1-[2-(6-méthoxy-naphtalén-2-yl)-propionyl]-pyrrolidine-2-carbonyl}-amino-3-mercapto-propionique

5. Composé 9 ayant la formule :
ester éthylique de l'acide 3-mercapto-2-({1-[2-(6-méthoxy-naphtalén-2-yl)-propionyl]-pipéridine-2-carbonyl}-amino-propionique

6. Composé 1 ayant la formule :
(2-mercapto-éthyl)amide de l'acide 1-{2-[1-(4-chlorobenzoyl)-5-méthoxy-2-méthyl-1 H-indol-3-yl]-acétyl}-pyrrolidine-2-carboxylique.

7. Composé 4 ayant la formule :
(2-mercapto-éthyl)amide de l'acide 1-[2-(6-méthoxy-naphtalén-2-yl)-propionyl]-pyrrolidine-2-carboxylique.

8. Composé 3 ayant la formule :
ester 2-méthoxy-4-méthylphénylique de l'acide 1-{2-[1-(4-chloro-benzoyl)-5-méthoxy-2-méthyl-1H-indol-3-yl]-acétyl}-pyrrolidine-2-carboxylique.

9. Composé 6 ayant la formule :
ester 2-méthoxy-4-méthylphénylique de l'acide 1-[2-(6-méthoxy-naphtalén-2-yl)-propionyl]-pyrrolidine-2-carboxylique.

10. Composé 7 ayant la formule :
(2-mercapto-éthyl)amide de l'acide 4-hydroxy-1-[2-(6-méthoxy-naphtalén-2-yl)-propionyl]-pyrrolidine-2-carboxylique.

11. Utilisation de l'un quelconque des composés selon l'une quelconque des revendications 1 à 10, pour la préparation d'un médicament pour le traitement de la maladie d'Alzheimer ou d'autres troubles neurodégénératifs ou pour le ralentissement de la progression de la maladie d'Alzheimer ou d'autres troubles neurodégénératifs ou pour le traitement de cas dans lesquels une inflammation ou un stress oxydatif du SNC est diagnostiqué(e) ou suspecté(e).

12. Composition pharmaceutique contenant en tant que principe actif au moins un composé de la formule générale I selon la revendication 1 ou l'un des composés 1 à 9 selon les revendications 2 à 10.

13. Procédé de préparation des composés 1 à 3 (dérivés de l'indométhacine) :

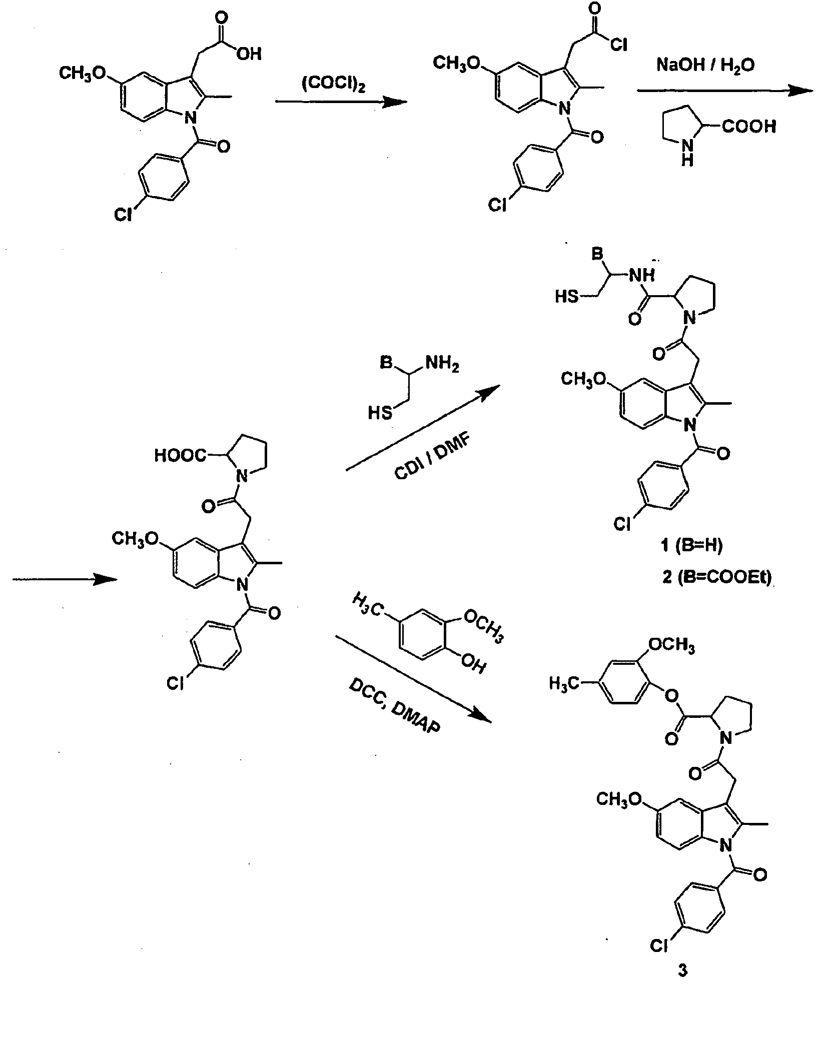

14. Procédé de préparation des composés 4 à 9 (dérivés du naproxène) :
